(19) Europäisches Patentamt
European Patent Office
Office européen des brevets

(11) Veröffentlichungsnummer: **0 643 060 A2**

# (12) EUROPÄISCHE PATENTANMELDUNG

(21) Anmeldenummer: **94112023.0**

(51) Int. Cl.[6]: **C07D 403/12**, A61K 31/41

(22) Anmeldetag: **02.08.94**

(30) Priorität: **13.08.93 DE 4327256**

(43) Veröffentlichungstag der Anmeldung:
**15.03.95 Patentblatt 95/11**

(84) Benannte Vertragsstaaten:
**AT BE CH DE DK ES FR GB GR IE IT LI LU MC NL PT SE**

(71) Anmelder: **BAYER AG**

**D-51368 Leverkusen (DE)**

(72) Erfinder: **Hanko, Rudolf, Dr.**
**Waldsaum 25**
**D-45134 Essen (DE)**
Erfinder: **Krämer, Thomas, Dr.**
**Schneewittchenweg 37**
**D-42111 Wuppertal (DE)**
Erfinder: **Dressel, Jürgen, Dr.**
**Tuchstrasse 48**
**D-42477 Radevormwald (DE)**
Erfinder: **Fey, Peter, Dr.**
**Am Eickhof 23**
**D-42111 Wuppertal (DE)**
Erfinder: **Hübsch, Walter, Dr.**
**Wildsteig 22**
**D-42113 Wuppertal (DE)**
Erfinder: **Müller, Ulrich, Dr.**
**Neuer Triebel 91**
**D-42111 Wuppertal (DE)**
Erfinder: **Müller-Gliemann, Matthias, Dr.**
**Laibacher Strasse 10**
**D-42697 Solingen (DE)**
Erfinder: **Beuck, Martin, Dr.**
**Trills 7**
**D-40699 Erkrath (DE)**
Erfinder: **Kazda, Stanislav, Prof. Dr.**
**Gellertweg 18**
**D-42115 Wuppertal (DE)**
Erfinder: **Knorr, Andreas, Dr.**
**Trillser Graben 10**
**D-40699 Erkrath (DE)**
Erfinder: **Stasch, Johannes-Peter, Dr.**
**Alfred-Nobel-Strasse 109**
**D-42651 Solingen (DE)**
Erfinder: **Wohlfeil, Stefan, Dr.**
**Tucherweg 25**
**D-40724 Hilden (DE)**
Erfinder: **Hirth-Dietrich, Claudia, Dr.**
**Stockmannsmühle 127**
**D-42115 Wuppertal (DE)**

(54) **Sulfonylbenzyl-substituierte Benzimidazole, Verfahren zu ihrer Herstellung und ihre Verwendung in Arzneimitteln.**

(57) Die vorliegende Erfindung betrifft neue Sulfonylbenzyl-substituierte Benzimidazole der allgemeinen Formel (I)

EP 0 643 060 A2

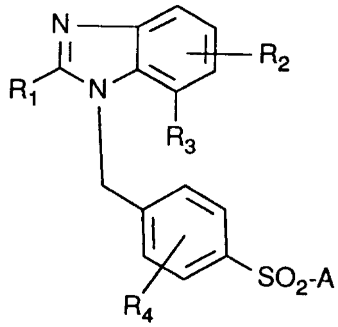

(I),

in welcher $R_1$, $R_2$, $R_3$, $R_4$ und A die in der Beschreibung angegebene Bedeutung haben, ein Verfahren zu ihrer Herstellung und ihre Verwendung in Arzneimitteln, insbesondere als blutdrucksenkende und anti-atherosklerotische Mittel.

Die vorliegende Erfindung betrifft neue Sulfonylbenzyl-substituierte Benzimidazole, ein Verfahren zu ihrer Herstellung und ihre Verwendung in Arzneimitteln, insbesondere als blutdrucksenkende und anti-atherosklerotische Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksteigerndes Oktapeptid Angiotensin II abgebaut wird. Die verschiedenen Effekte des Angiotensin II, wie beispielsweise Vasokonstriktion, $Na^+$-Retention in der Niere, Aldosteronfreisetzung in der Nebenniere und Tonuserhöhung des symphathischen Nervensystems wirken synergistisch im Sinne einer Blutdruckerhöhung.

Darüberhinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

Aus der PCT WO 92/03423 sind Benzimidazole mit einer PAF (platelet activating factor) antagonistischen Wirkung bekannt, wobei die erfindungsgemäßen Verbindungen von dem breiten allgemeinen Bedeutungsumfang dieser Publikation erfaßt werden, ohne dort ausdrücklich genannt zu werden.

Die vorliegende Erfindung betrifft ausgewählte neue Sulfonylbenzyl-substituierte Benzimidazole der allgemeinen Formel (I)

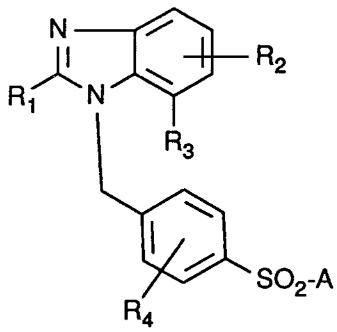

(I),

in welcher

$R^1$ für geradkettiges oder verzweigtes Alkyl mit bis zu 8 Kohlenstoffatomen steht, das gegebenenfalls durch Cycloalkyl mit 3 bis 6 Kohlenstoffatomen, Halogen, Hydroxy, Phenyl oder durch geradkettiges oder verzweigtes Alkoxy mit bis zu 6 Kohlenstoffatomen substituiert ist, oder
für Cycloalkyl mit 3 bis 8 Kohlenstoffatomen steht, oder
für eine Gruppe der Formel $-OR^5$ steht, worin

$R^5$ geradkettiges oder verzweigtes Alkyl mit bis zu 8 Kohlenstoffatomen, Cycloalkyl mit 3 bis 7 Kohlenstoffatomen, Aryl mit 6 bis 10 Kohlenstoffatomen oder einen 5- bis 7-gliedrigen, gesättigten oder ungesättigten Heterocyclus mit bis zu 4 Heteroatomen aus der Reihe S, N oder O bedeutet,

$R^2$ für Wasserstoff, Halogen oder für geradkettiges oder verzweigtes Alkyl oder Alkenyl mit jeweils bis zu 8 Kohlenstoffatomen steht, die gegebenenfalls durch Cycloalkyl mit 3 bis 6 Kohlenstoffatomen substituiert sind, oder
für Cycloalkyl mit 3 bis 8 Kohlenstoffatomen steht, oder
für geradkettiges oder verzweigtes Perfluoralkyl mit bis zu 6 Kohlenstoffatomen steht,

$R^3$ für einen Rest der Formel
$-CO-R^6$ , $-SO_3H$ ,

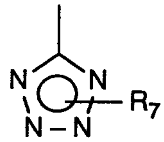

oder $-PO_3H$ steht,
worin

$R^6$ Hydroxy oder geradkettiges oder verzweigtes Alkoxy mit bis zu 8 Kohlenstoffatomen bedeutet, das gegebenenfalls durch Halogen, Carboxy, Hydroxy, Cyano oder durch geradkettiges oder verzweigtes Alkoxy oder Alkoxycarbonyl mit jeweils bis zu 6 Kohlenstoffatomen substituiert ist, oder
Aryloxy mit 6 bis 10 Kohlenstoffatomen oder Heterocyclyloxy bedeutet, oder
Cycloalkyloxy mit 3 bis 7 Kohlenstoffatomen bedeutet, oder eine Gruppe der Formel $-NR^8R^9$ bedeutet, worin

$R^8$ und $R^9$ gleich oder verschieden sind und Wasserstoff oder geradkettiges oder verzweigtes Alkyl mit bis zu 6 Kohlenstoffatomen oder Phenyl bedeuten,

$R^7$ Wasserstoff, geradkettiges oder verzweigtes Alkyl mit bis zu 6 Kohlenstoffatomen oder Triphenylmethyl bedeutet

$R^4$ für Wasserstoff, Halogen, geradkettiges oder verzweigtes Alkyl mit bis zu 8 Kohlenstoffatomen steht, oder
für geradkettiges oder verzweigtes Perfluoralkyl mit bis zu 6 Kohlenstoffatomen steht, oder
für eine Gruppe der Formel $-OR^{10}$ oder $-SO_2-NR^{11}R^{12}$ steht, worin

$R^{10}$ die oben angegebene Bedeutung von $R^5$ hat und mit dieser gleich oder verschieden ist, oder eine Hydroxyschutzgruppe oder Wasserstoff bedeutet,

$R^{11}$ und $R^{12}$ gleich oder verschieden sind und Wasserstoff, Cycloalkyl mit 3 bis 7 Kohlenstoffatomen, Aryl mit 6 bis 10 Kohlenstoffatomen oder einen 5- bis 7-gliedrigen, gesättigten oder ungesättigten Heterocyclus mit bis zu 3 Heteroatomen aus der Reihe S, N oder O bedeuten, oder geradkettiges oder verzweigtes Alkyl mit bis zu 6 Kohlenstoffatomen bedeuten, das gegebenenfalls durch Halogen, Carboxy, Hydroxy, Cyano oder durch geradkettiges oder verzweigtes Alkoxy oder Alkoxycarbonyl mit jeweils bis zu 6 Kohlenstoffatomen substituiert ist, oder

$R^{11}$ und $R^{12}$ gemeinsam unter Einbezug des Stickstoffatoms einen 5- bis 7-gliedrigen, gesättigten oder ungesättigten heterocyclischen Ring mit bis zu 3 Heteroatomen aus der Reihe S, N oder O bilden,

A für einen über das Stickstoffatom gebundenen 3 bis 8-gliedrigen, gesättigten Heterocyclus mit bis zu 2 weiteren Heteroatomen aus der Reihe S, N oder O steht und der gegebenenfalls bis zu 2-fach gleich oder verschieden durch einen Rest der Formel
$-SO_3H$ ,

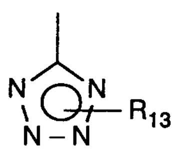 ,

$-CO-R^{14}$ oder $-PO(OR^{15})(OR^{16})$
substituiert ist, worin

$R^{13}$ die oben angegebene Bedeutung von $R^7$ hat und mit dieser gleich oder verschieden ist und

$R^{14}$ die oben angegebene Bedeutung von $R^6$ hat und mit dieser gleich oder verschieden ist,

$R^{15}$ und $R^{16}$ gleich oder verschieden sind und
Wasserstoff, geradkettiges oder verzweigtes Alkyl mit bis zu 8 Kohlenstoffatomen oder Phenyl bedeuten

und deren Salze.

Die erfindungsgemäßen Sulfonylbenzyl-substituierten Benzimidazole können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der Sulfonylbenzyl-substituierten Benzimidazole können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure, Trifluoressigsäure oder Benzoesäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium- oder Calciumsalze, sowie Ammoniumsalze, die abgeleitet sind von Ammoniak oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, entweder als Enantiomer oder als Diastereomer, existieren. Die Erfindung betrifft sowohl die Enantiomeren oder Diastereomeren als auch deren jeweiligen Mischungen. Die Racemformen lassen sich ebenso wie die Diastereomeren in bekannter Weise in die stereoisomer einheitlichen Bestandteile trennen.

Ein über N-gebundener, 3- bis 8-gliedriger gesättigter Heterocyclus, der außerdem als Heteroatome bis zu 2 Sauerstoff-, Schwefel- und/oder Stickstoffatome enthalten kann, steht im allgemeinen für Azetidinyl, Piperidyl, Morpholinyl, Piperazinyl oder Pyrrolidinyl. Bevorzugt sind 5- und 6-gliedrige Ringe mit einem Sauerstoff- und/oder bis zu 2-Stickstoffatomen, wie beispielsweise Azetidinyl, Piperidyl, Morpholinyl oder Piperazin oder Pyrrolidinyl. Besonders bevorzugt sind Piperidyl und Pyrrolidinyl.

Ein 5- bis 7-gliedriger Heterocyclus mit bis zu 4 Heteroatomen aus der Reihe S, N oder O steht im Rahmen der Erfindung beispielsweise für Imidazolyl, Isothiazolyl, Tetrazolyl, Thiazolyl, Thiazolinyl, Isoxazolyl, 1,2,4-Triazolyl, 1,3,4-Thiadiazolyl, Pyridimidyl, Pyrazinyl, Pyrryl, Thienyl oder Furyl.

Hydroxyschutzgruppe im Rahmen der oben angegebenen Definition steht im allgemeinen für eine Schutzgruppe aus der Reihe: Trimethylsilyl, Triethylsilyl, Triisopropylsilyl, tert.Butyldimethylsilyl, tert.Butyldiphenylsilyl, Triphenylsilyl, Trimethylsilylethoxycarbonyl, Benzyl, Triphenylmethyl (Trityl), Monomethoxytrityl (MMTr), Dimethoxytrityl (DMTr), Benzyloxycarbonyl, 2-Nitrobenzyl, 4-Nitrobenzyl, 2-Nitrobenzyloxycarbonyl, 4-Nitrobenzyloxycarbonyl, tert.Butyloxycarbonyl, Allyloxycarbonyl, 4-Methoxybenzyl, 4-Methoxybenzyloxycarbonyl, Formyl, Acetyl, Trichloracetyl, 2,2,2-Trichlorethoxycarbonyl, 2,4-Dimethoxybenzyl, 2,4-Dimethoxybenzyloxycarbonyl, Methoxymethyl, Methylthiomethyl, Methoxyethoxymethyl, [2-(Trimethylsilyl)ethoxy]methyl, 2-(Methylthiomethoxy)-ethoxycarbonyl, Tetrahydropyranyl, Benzoyl, 4-Methylbenzoyl, 4-Nitrobenzoyl, 4-Fluorbenzoyl, 4-Chlorbenzoyl oder 4-Methoxybenzoyl. Bevorzugt sind Acetyl, Benzyl und tert.Butyldimethylsilyl.

Heterocyclyloxy steht im Rahmen der Erfindung für einen der oben unter den Heterocyclen angegebenen Ringe, wobei dieser jeweils über Sauerstoff gebunden wird.

Bevorzugt sind Verbindungen der allgemeinen Formel (I), in welcher

$R^1$ für geradkettiges oder verzweigtes Alkyl mit bis zu 6 Kohlenstoffatomen steht, das gegebenenfalls durch Cyclopropyl, Cyclobutyl, Cyclopentyl, Cyclohexyl Fluor, Chlor, Brom, Hydroxy, Phenyl oder durch geradkettiges oder verzweigtes Alkoxy mit bis zu 4 Kohlenstoffatomen substituiert ist, oder
für Cyclopropyl, Cyclopentyl oder Cyclohexyl steht, oder
für eine Gruppe der Formel $-OR^5$ steht, worin

$R^5$ geradkettiges oder verzweigtes Alkyl mit bis zu 6 Kohlenstoffatomen, Cyclopentyl, Cyclohexyl, Cycloheptyl oder Phenyl bedeutet

$R^2$ für Wasserstoff, Halogen oder für geradkettiges oder verzweigtes Alkyl oder Alkenyl mit jeweils bis zu 6 Kohlenstoffatomen steht, die gegebenenfalls durch Cyclopropyl, Cyclobutyl, Cyclopentyl oder Cyclohexyl substituiert sind, oder
für Cyclopropyl, Cyclopentyl oder Cyclohexyl steht, oder
für geradkettiges oder verzweigtes Perfluoralkyl mit bis zu 4 Kohlenstoffatomen steht,

$R^3$ für einen Rest der Formel
$-CO-R^6$ , $-SO_3H$ ,

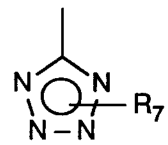

oder $-PO_3H$ steht,
worin

$R^6$ Hydroxy oder geradkettiges oder verzweigtes Alkoxy mit bis zu 6 Kohlenstoffatomen bedeutet, das gegebenenfalls durch Fluor, Chlor, Brom, Carboxy, Hydroxy, Cyano oder durch geradkettiges oder verzweigtes Alkoxy oder Alkoxycarbonyl mit jeweils bis zu 4 Kohlenstoffatomen substituiert ist, oder Phenoxy, Cyclopentyloxy oder Cyclohexyloxy bedeutet, oder eine Gruppe der Formel $-NR^8R^9$ bedeutet, worin

$R^8$ und $R^9$ gleich oder verschieden sind und Wasserstoff oder geradkettiges oder verzweigtes Alkyl mit bis zu 4 Kohlenstoffatomen bedeuten,

$R^7$ Wasserstoff, geradkettiges oder verzweigtes Alkyl mit bis zu 4 Kohlenstoffatomen oder Triphenylmethyl bedeutet

$R^4$ für Wasserstoff, Fluor, Chlor, Brom, geradkettiges oder verzweigtes Alkyl mit bis zu 6 Kohlenstoffatomen, oder für geradkettiges oder verzweigtes Perfluoralkyl mit bis zu 4 Kohlenstoffatomen steht, oder eine Gruppe der Formel $-OR^{10}$ oder $-SO_2NR^{11}R^{12}$ steht, worin

$R^{10}$ die oben angegebene Bedeutung von $R^5$ hat und mit dieser gleich oder verschieden ist, oder Benzyl oder Wasserstoff bedeutet,

$R^{11}$ und $R^{12}$ gleich oder verschieden sind und Wasserstoff, Cyclopropyl, Cyclopentyl, Cyclohexyl, Phenyl bedeuten, oder geradkettiges oder verzweigtes Alkyl mit bis zu 5 Kohlenstoffatomen bedeuten, das gegebenenfalls durch Fluor, Chlor, Brom, Carboxy, Hydroxy, Cyano oder durch geradkettiges oder verzweigtes Alkoxy oder Alkoxycarbonyl mit jeweils bis zu 4 Kohlenstoffatomen substituiert ist, oder

$R^{11}$ und $R^{12}$ gemeinsam unter Einbezug des Stickstoffatoms einen Morpholinring bilden,

A für über das Stickstoffatom gebundenes Azetidinyl, Piperidyl, Pyrrolidinyl oder Morpholinyl steht, die gegebenenfalls durch einen Rest der Formel
$-SO_3H$ ,

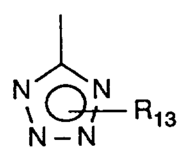 ,

$-CO-R^{14}$ oder $-PO(OR^{15})(OR^{16})$
substituiert sind, worin

$R^{13}$ die oben angegebene Bedeutung von $R^7$ hat und mit dieser gleich oder verschieden ist,

$R^{14}$ die oben angegebene Bedeutung von $R^6$ hat und mit dieser gleich oder verschieden ist,

$R^{15}$ und $R^{16}$ gleich oder verschieden sind und Wasserstoff, geradkettiges oder verzweigtes Alkyl mit bis zu 6 Kohlenstoffatomen oder Phenyl bedeuten

und deren Salze.

Besonders bevorzugt sind Verbindungen der allgemeinen Formel (I), in welcher

$R^1$ für geradkettiges oder verzweigtes Alkyl mit bis zu 4 Kohlenstoffatomen steht, das gegebenenfalls durch Fluor, Chlor, Brom, Hydroxy, Phenyl oder durch geradkettiges oder verzweigtes Alkoxy mit bis zu 3 Kohlenstoffatomen substituiert ist, oder für Cyclopropyl, Cyclopentyl oder Cyclohexyl steht, oder für eine Gruppe der Formel $-OR^5$ steht, worin

$R^5$ geradkettiges oder verzweigtes Alkyl mit bis zu 5 Kohlenstoffatomen, Cyclopentyl, Cyclohexyl, Cycloheptyl, Phenyl steht,

| | |
|---|---|
| $R^2$ | für Wasserstoff, Fluor, Chlor, Brom oder für geradkettiges oder verzweigtes Alkyl oder Alkenyl mit jeweils bis zu 4 Kohlenstoffatomen oder Cyclopropyl steht, oder für geradkettiges oder verzweigtes Perfluoralkyl mit bis zu 3 Kohlenstoffatomen steht, |
| $R^3$ | für einen Rest der Formel $-CO-R^6$ , $-SO_3H$ , 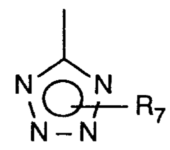 oder $-PO_3H$ steht, worin |
| $R^6$ | Hydroxy oder Alkoxy mit bis zu 4 Kohlenstoffatomen, Phenoxy oder Cyclopentyloxy oder Cyclohexyloxy bedeutet, oder eine Gruppe der Formel $-NR^8R^9$ bedeutet, worin |
| $R^8$ und $R^9$ | gleich oder verschieden sind und Wasserstoff oder geradkettiges oder verzweigtes Alkyl mit bis zu 3 Kohlenstoffatomen bedeuten, |
| $R^7$ | Wasserstoff, Methyl, Ethyl oder Triphenylmethyl bedeutet, |
| $R^4$ | für Wasserstoff, Fluor, Chlor, geradkettiges oder verzweigtes Alkyl mit bis zu 4 Kohlenstoffatomen, oder für geradkettiges oder verzweigtes Perfluoralkyl mit bis zu 3 Kohlenstoffatomen steht, oder für eine Gruppe der Formel $-OR^{10}$ oder $-SO_2NR^{11}R^{12}$ steht, worin |
| $R^{10}$ | die oben angegebene Bedeutung von $R^5$ hat und mit dieser gleich oder verschieden ist, oder Benzyl oder Wasserstoff bedeutet, |
| $R^{11}$ und $R^{12}$ | gleich oder verschieden sind und Wasserstoff, Cyclopropyl, Cyclopentyl, Cyclohexyl, Phenyl oder geradkettiges oder verzweigtes Alkyl mit bis zu 4 Kohlenstoffatomen bedeuten, |
| A | für über das Stickstoffatom gebundenes Azetidinyl, Piperidyl oder Pyrrolidinyl steht, die gegebenenfalls durch einen Rest der Formel $-SO_3H$ , 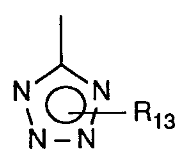 , $-CO-R^{14}$ oder $-PO(OR^{15})(OR^{16})$ substituiert ist, worin |
| $R^{13}$ | die oben angegebene Bedeutung von $R^7$ hat und mit dieser gleich oder verschieden ist, |
| $R^{14}$ | die oben angegebene Bedeutung von $R^6$ hat und mit dieser gleich oder verschieden ist, |
| $R^{15}$ und $R^{16}$ | gleich oder verschieden sind und Wasserstoff, geradkettiges oder verzweigtes Alkyl mit bis zu 4 Kohlenstoffatomen oder Phenyl bedeuten |

und deren Salze.

Außerdem wurde ein Verfahren zur Herstellung der erfindungsgemäßen Verbindungen der allgemeinen Formel (I) gefunden, dadurch gekennzeichnet,
daß man Sulfonylbenzylverbindungen der allgemeinen Formel (II)

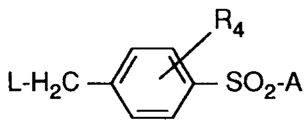 (II)

in welcher

A und $R^4$ die oben angegebene Bedeutung haben und

L für Halogen, vorzugsweise für Brom steht,

mit Nitrobenzoesäureestern der allgemeinen Formel (III)

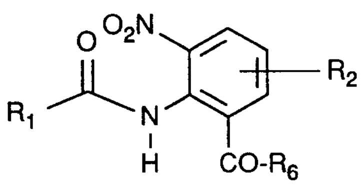 (III)

in welcher

$R^1$, $R^2$ und $R^6$ die oben angegebene Bedeutung haben,

zunächst in inerten Lösemitteln, in Anwesenheit einer Base umsetzt,

und anschließend in Gegenwart von Eisenpulver und Säure cyclisiert und gegebenenfalls die Substituenten $R^1$, $R^2$, $R^3$ und $R^4$ nach üblichen Methoden, beispielsweise durch Alkylierung oder Verseifung, variiert.

Das erfindungsgemäße Verfahren kann durch folgendes Formelschema beispielhaft erläutert werden:

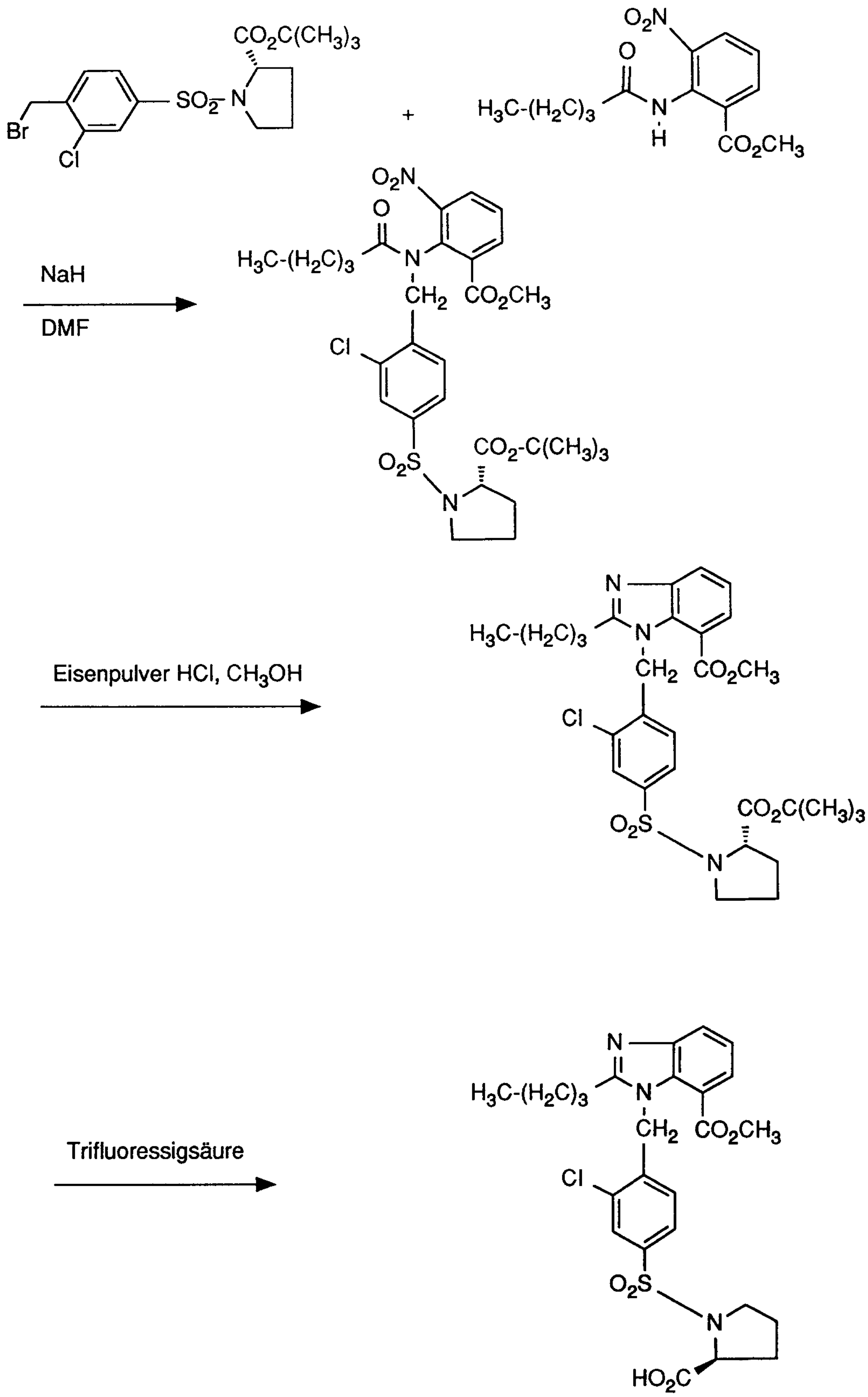


Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfrakionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind für die verschiedenen Schritte Tetrahydrofuran, Methylenchlorid, Toluol oder Dimethylformamid.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate oder -amide wie Natrium- oder Kaliummethanolat, Natrium- oder Kaliumethanolat oder Kalium-tert.butylat oder Lithiumdiisopropylamid (LDA), oder organische Amine (Trialkyl($C_1$-$C_6$)amine) wie Triethylamin, oder Heterocyclen wie 1,4-Diazabicyclo-[2.2.2]octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle, wie Natrium oder deren Hydride wie Natriumhydrid, einzusetzen. Bevorzugt sind Natriumhydrid, Lithiumdiisopropylamid (LDA) und DBU.

Im allgemeinen setzt man die Base in einer Menge von 0,05 mol bis 10 mol, bevorzugt von 1 mol bis 2 mol bezogen auf 1 mol der Verbindung der Formel (III) ein.

Das erfindungsgemäße Verfahren wird im allgemeinen in einem Temperaturbereich von -100 °C bis +100 °C, bevorzugt von 0 °C bis +30 °C durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung kann auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0 °C bis +100 °C, bevorzugt von +20 °C bis +80 °C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters, eingesetzt. Besonders bevorzugt verwendet man molare Mengen der Reaktanden.

Bei der Durchführung der Reaktion entstehen im ersten Schritt die Carboxylate der erfindungsgemäßen Verbindungen als Zwischenprodukte, die isoliert werden können. Die erfindungsgemäßen Säuren erhält man durch Behandeln der Carboxylate mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren hierbei als vorteilhaft erwiesen, die basische Reaktionsmischung der Verseifung in einem zweiten Schritt ohne Isolierung der Carboxylate anzusäuern. Die Säuren können dann in üblicher Weise isoliert werden. Im Fall der basischen Heterocyclen können durch das Behandeln der Lösungen der Carboxylate mit den oben aufgeführten Säuren auch die Salze der Heterocyclen mit den anorganischen Säuren gewonnen werden.

Die Alkylierung erfolgt im allgemeinen mit Alkylierungsmitteln wie beispielsweise ($C_1$-$C_8$)-Alkylhalogeniden, Sulfonsäureestern oder substituierten oder unsubstituierten ($C_1$-$C_8$)-Dialkyl- oder ($C_1$-$C_8$)-Diarylsulfonate, vorzugsweise Methyljodid oder Dimethylsulfat.

Die Alkylierung erfolgt im allgemeinen in einem der oben aufgeführten Lösemitteln, vorzugsweise in Dimethylformamid in einem Temperaturbereich von 0 °C bis +70 °C, vorzugsweise von 0 °C bis +30 °C und Normaldruck.

Die Verbindungen der allgemeinen Formel (II) sind neu und können beispielsweise hergestellt werden, indem man im Fall, daß $R^4 \neq -OR^{10}$ ($R^{4'}$), Verbindungen der allgemeinen Formel (IV)

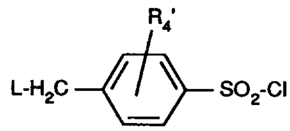 (IV)

in welcher

L und $R^{4'}$ die oben angegebene Bedeutung haben

mit Verbindungen der allgemeinen Formel (V)

A-H (V)

in welcher

A die oben angegebene Bedeutung hat,

in einem der oben aufgeführten Lösemitteln in Basen, vorzugsweise in Dichlormethan mit Triethylamin bei Normaldurck und in einem Temperaturbereich von -10 °C bis +120 °C, vorzugsweise bei 0 °C umsetzt und im Fall, daß $R^4$ für die Gruppe der Formel $-OR^{10}$ steht,

ausgehend von Carboxy, Hydroxy-disubstituierten Benzolsulfonsäurechloriden, beispielsweise 4-Carboxy-3-hydroxybenzolsulfochlorid, zunächst durch Umsetzung mit Verbindungen der allgemeinen Formel (V), Verbindungen der allgemeinen

Formel (VI) ($R^{10} = H \triangleq R^{4''}$) ,

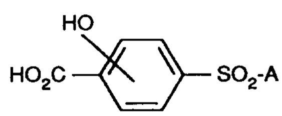 (VI)

in welcher

A die oben angegebene Bedeutung hat,

herstellt,

anschließend durch Überführung der Carboxyfunktion in den entsprechendn Benzylester und der Blockierung der Hydroxyfunktion nach üblichen Methoden in die Verbindungen der allgemeinen Formel (VII)

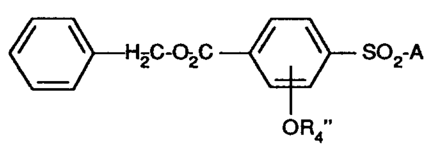 (VII)

in welcher

A und $R^{4''}$ die oben angegebene Bedeutung haben,

überführt,

in einem weiteren Schritt, ebenfalls nach bekannten Methoden, vorzugsweise mit Natriumborhydrid/LiCl in Diglyme den Benzylester zur Hydroxymethylfunktion reduziert,

und abschließend mit Triphenylphosphindibromid, in einem geeigneten Lösemittel, vorzugsweise Acetonitril, unter Schutzgasatmosphäre, in einem Temperaturbereich von 0 °C bis Raumtemperatur, bromiert.

Die Verbindungen der allgemeinen Formeln (VI) und (VII) sind an sich neu und können wie oben beschrieben hergestellt werden.

Die Verbindungen der allgemeinen Formeln (IV) und (V) sind bekannt oder können nach üblicher Methode hergestellt werden.

Die Verbindungen der allgemeinen Formel (III) sind ebenfalls an sich bekannt.

Die vorstehenden Herstellungsverfahren sind lediglich zur Verdeutlichung angegeben. Die Herstellung der erfindungsgemäßen Verbindungen der allgemeinen Formel (I) ist nicht auf diese Verfahren beschränkt,

jede Modifikation dieser Verfahren ist in gleicher Weise für die Herstellung anwendbar.

Die erfindungsgemäßen Verbindungen der allgemeinen Formel (I) zeigen ein nicht vorhersehbares, wertvolles pharmakologisches Wirkspektrum.

Die erfindungsgemäßen Verbindungen besitzen eine spezifische A II-antagonistische Wirkung, da sie kompetitiv die Bindung von Angiotensin II an die Rezeptoren hemmen. Sie unterdrücken die vasokonstriktorischen und Aldosteronsekretionsstimulierenden Effekte des Angiotensin II. Darüberhinaus inhibieren sie die Proliferation von glatten Muskelzellen.

Sie können deshalb in Arzneimittel zur Behandlung der arteriellen Hypertonie und Atherosklerose eingesetzt werden. Darüberhinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden. Darüberhinaus besitzen die Substanzen natriuretische und diuretische Wirkung. Diese Wirkung äußert sich in einer Ödemausschwemmung bei pathologischer Flüssigkeitsvermehrung kardialen und nicht kardialen Ursprungs.

Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37°C temperierter, Carbogenbegaster Krebs-Henseleit-Nährlösung folgender Zusammensetzung: 119 mmol/l NaCl; 2,5 mmol/l $CaCl_2$ x 2 $H_2O$; 1,2 mmol/l $KH_2PO_4$; 10 mmol/l Glucose; 4,8 mmol/l KCl; 1,4 mmol/l $MgSO_4$ x 7 $H_2O$ und 25 mmol/l $NaHCO_3$ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

Agonisten und ihre Standardkonzentrationen Applikationsvolumen pro Einzelgabe = 100 µl):

| | | |
|---|---|---|
| KCl | 22,7;32,7;42,7;52,7 | mmol/l |
| Noradrenalin | $3x10^{-9}$;$3x10^{-8}$;$3x10^{-7}$;$3x10^{-6}$ | g/ml |
| Serotonin | $10^{-8}$;$10^{-7}$;$10^{-6}$;$10^{-5}$ | g/ml |
| B-HT 920 | $10^{-7}$;$10^{-6}$;$10^{-5}$ | g/ml |
| Methoxamin | $10^{-7}$;$10^{-6}$;$10^{-5}$ | g/ml |
| Angiotensin II | $3x10^{-9}$;$10^{-8}$;$3x10^{-8}$;$10^{-7}$ | g/ml |

Für die Berechnung der $IC_{50}$ (Konzentration bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

Tabelle A:

Hemmung der Gefäßkontraktion an isolierten Aortenringen von Kaninchen in vitro

$IC_{50}$ [g/ml] gegen Kontraktionen, induziert durch AII:

| Bsp.-Nr. | $IC_{50}$[nM] |
|---|---|
| 2 | 46 |
| 6 | 6,4 |

Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht.

Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft. Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht.
Der arterielle Blutdruck dieser Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

Interaktion der erfindungsgemäßen Verbindungen mit dem Angiotensin II-Rezeptor an Membranfraktionen der Nebennierenrinde (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.
Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 µg), $^{3}H$-Angiotensin II (3-5 nM), Test-Pufferlösung (50 mM Tris, pH 7,2, 5 mM $MgCl_2$ sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu $K_i$- bzw. $IC_{50}$-Werten ($K_i$: für die verwendete Radioaktivität korrigierte $IC_{50}$-Werte; $IC_{50}$-Werte: Konzentration bei der die zu untersuchende Substanz eine 50%ige Hemmung der spezifischen Bindung des Radioliganden bewirkt).

Tabelle B:

| Bsp.-Nr. | $K_i$ [nM] |
|---|---|
| 3 | 48 |
| 4 | 735 |
| 7 | 260 |

Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. 50, 172, 1971]. Die Zellen werden in geeigneten Kulturschalen, in der Regel 96-Loch-Platten, ausgesät und für 2 - 3 Tage in Medium 199 mit 7,5% FCS und 7,5% NCS, 2 mM L-Glutamin und 15 mM HEPES, pH 7,4 in 5% $CO_2$ bei 37°C kultiviert. Danach werden die Zellen durch Serumentzug für 2 - 3 Tage synchronisiert und sodann mit Serum oder anderen Faktoren zum Wachstum angeregt. Gleichzeitig werden Testverbindungen zugesetzt. Nach 16 - 20 Stunden wird 1 µCi $^3$H-Thymidin zugefügt und nach weiteren 4 Stunden der Einbau dieser Substanz in die TCA-präzipitierbare DNA der Zellen bestimmt. Zur Bestimmung der $IC_{50}$-Werte wird die Wirkstoffkonzentration errechnet, die bei sequentieller Verdünnung des Wirkstoffes halbmaximale Hemmung der durch 10% FCS hervorgerufene Thymidininkorporation bewirkt.

Prüfung auf natriuretische Wirkung

Nüchterne Wistar-Ratten werden mit Prüfsubstanz (suspendiert in Tylose-Lösung) oral behandelt. Anschließend wird in Diurese-Käfigen die Harnausscheidung über 6 Stunden gesammelt. Die Konzentration von Natrium und Kalium im Harn wird flammenphotometrisch bestimmt.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

| Laufmittelgemische: | |
|---|---|
| a = Methylenchlorid/Methanol | 10:1 |
| b = Petrolether/Essigester | 1:1 |
| c = Essigester/Petrolether | 2:1 |
| d = Methylenchlorid/Methanol | 4:1 |

Ausgangsverbindungen

Beispiel I

4-(Brommethyl)benzol-sulfochlorid

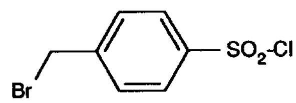

38,1 g (0,2 mol) 4-Methylbenzolsulfonylchlorid wurden in 300 mi Tetrachlorkohlenstoff gelöst und mit 35,6 g (0,2 mol) N-Bromsuccinimid versetzt und nach Zugabe von 0,2 g (1,2 mmol) Azobisisobutyronitril (ABU) für 4 h unter Rückfluß erhitzt. Nach dem Abkühlen wurden die Feststoffe abfiltriert und das Filtrat vom Lösemittel befreit. Flash-Chromatographie (Petrolether / Toluol 4:1, 50 µm Korngröße) und anschließende Umkristallisation aus 100 ml Cyclohexan ergab 24,0g (45% der Theorie) der Titelverbindung.
$R_f$ = 0,75 (Toluol)

Beispiel II

4-(Brommethyl)-3-chlorbenzolsulfochlorid

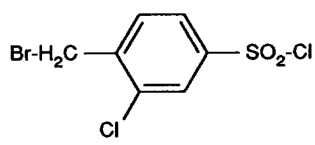

45,9 g (0,2 mol) 3-Chlor-4-methylbenzolsulfonsäure Natriumsalz wurden mit 83,3 g (0,4 mol) Phosphorpentachlorid gemischt und 30 min bei 140 °C Ölbadtemperatur erhitzt. In der Hitze wurde mit 500 ml Toluol versetzt, die entstandene Lösung bis zum Sieden erhitzt und nach dem Abkühlen auf Eis gegeben. Die organische Phase wurde abgetrennt und mit Wasser gewaschen (2 x 200 ml). Nach dem Trocknen über $MgSO_4$ wurde filtriert und alles Flüchtige im Vakuum abgezogen. Der erhaltene Rückstand wurde durch Flashchromatographie (Petrolether / Toluol 4:1, 50 µ Korngröße) gereinigt. Man erhält 24,9 g eines Produkts das sofort weiter umgesetzt wird:
Man nahm in 200 ml Tetrachlorkohlenstoff auf und erhitzte nach Zugabe von 19,6 g (0,11 mol) N-Bromsuccinimid und 0,1 g (0,6 mmol) ABN für 6 h unter Rückfluß. Nach dem Abkühlen wurden die Feststoffe abfiltriert und das Filtrat vom Lösemittel befreit. Flashchromatographie (Petrolether / Toluol 4:1, 50 µ Korngröße) ergaben 21,2 g (35%) der Titelverbindung.
$R_f$ = 0,32 (Petrolether / Dichlormethan 4:1)

Beispiel III

4-(Brommethyl)-3-fluorbenzolsulfochlorid

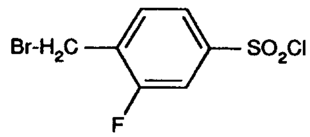

Man nahm 20,9 g (0,1 mol) 3-Fluor-4-methylbenzolsulfochlorid in 200 ml Tetrachlorkohlenstoff auf und erhitzte nach Zugabe von 19,6 g (0,11 mol) N-Bromsuccinimid und 0,3 g Dibenzoylperoxid für 5 h unter Rückfluß. Nach dem Abkühlen wurden die Feststoffe abfiltriert und das Filtrat vom Lösemittel befreit. Flashchromatographie Petrolether/Toluol (4:1). 50 µm Korngröße ergaben 12,4 g (44% d.Th.) der Titelverbindung.
$R_f$ = 0,42 (Petrolether/Toluol 3:1)

Beispiel IV

4-(Brommethyl)-benzolsulfonyl-N-pyrrolidinid

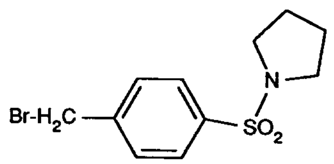

5,3 g (0,02 mol) der Verbindung aus Beispiel I wurden in 200 ml Dichlormethan und 4,0 g (0,04 mol) Triethylamin gelöst und nach Zugabe von 1,4 g (0,02 mol) Pyrrolidin in 50 ml Dichlormethan bei 0 °C für 1 h bei 0 °C nachgerührt. Man extrahierte mit 2 N HCl (2 x 100 ml), $H_2O$ (2 x 100 ml), trocknete über $MgSO_4$, filtrierte und verdampfte alle flüchtigen Anteile im Vakuum. Ausbeute: 5,4 g (89% der Theorie)
$R_f$ = 0,09 (Toluol)

Beispiel V

4-(Brommethyl)benzolsulfonyl-N-piperidinid

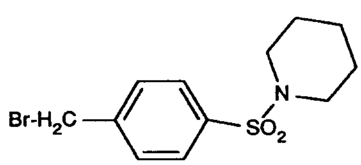

In Analogie zur Vorschrift des Beispiels III erhielt man aus 1,1 g (4 mmol) der Verbindung aus Beispiel I und 0,34 g (4 mmol) Piperidin 1,0 g (81% der Theorie) der Titelverbindung.
$R_f$ = 0,14 (Toluol)

Beispiel VI

(S)-4-(Brommethyl)-benzolsulfonyl-N-2-(tert.butoxycarbonyl)pyrrolidinid

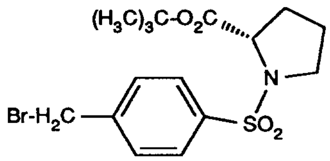

In Analogie zur Vorschrift des Beispiels III erhielt man aus 7,25 g (27 mmol) der Verbindung aus Beispiel I und 4,6 g (27 mmol) S-Prolin-tert.butylester 9,1 g (84% der Theorie) der Titelverbindung.
$R_f$ = 0,66 (Petrolether / Essigester 7:3)

Beispiel VII

rac-4-(Brommethyl)-benzolsulfonyl-N-2-(tert.butoxycarbonyl)piperidinid

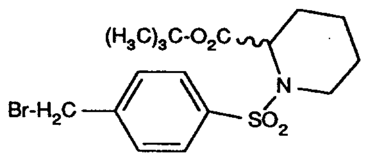

In Analogie zur Vorschrift des Beispiels III erhielt man aus 8,0 g (30 mmol) der Verbindung aus Beispiel I und 5,5 g (30 mmol) rac-Pipercolinsäure-tert.butylester 7,4 g (59% der Theorie) der Titelverbindung.
$R_f$ = 0,53 (Petrolether / Essigester 5:1)

Beispiel VIII

(S)-4-(Brommethyl)-3-chlorbenzolsulfonyl-N-2-(tert.butoxycarbonyl)pyrrolidinid

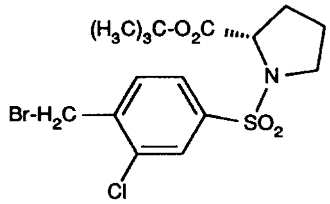

In Analogie zur Vorschrift des Beispiels III erhielt man aus 10,0 g (33 mmol) der Verbindung aus Beispiel II und 5,7 g (33 mmol) S-Prolin-tert.butylester 13,9 g (96% der Theorie) der Titelverbindung.
$R_f$ = 0,55 (Petrolether / Essigester 7:3)

Beispiel IX

rac-4-(Brommethyl)-3-chlorbenzolsulfonyl-N-2-(tert.butoxycarbonyl)piperidinid

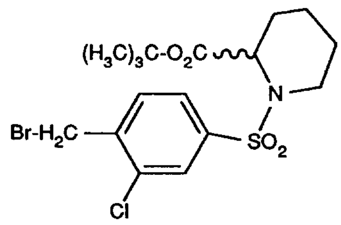

In Analogie zur Vorschrift des Beispiels III erhielt man aus 10,0 g (33 mmol) der Verbindung aus Beispiel II und 6,1 g (33 mmol) rac-Pipecolinsäure-tert.butylester 14,6 g (98% der Theorie) der Titelverbindung.
$R_f$ = 0,6 (Petrolether / Essigester 7:3)

Beispiel X

(S)-4-(Brommethyl)-3-fluorbenzolsulfonyl-N-2-(tert.butoxy-carbonyl)pyrrolidinid

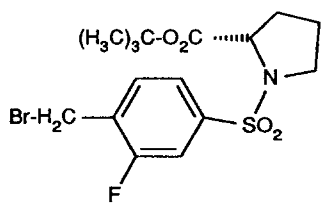

In Analogie zur Vorschrift aus Beispiel IV erhielt man aus 8,6 g (30 mmol) der Verbindung aus Beispiel III und 5,1 g (30 mmol) S-Prolin-tert.butylester 12,7 g (100% d.Th.) der Titelverbindung.
$R_f$ = 0,57 (Petrolether/Essigester 7:3)

Beispiel XI

(S)-2-[3-Chlor-1-(N-2-(tert.butyloxycarbonyl)pyrrolidinyl)sulfonyl-phen-4-yl-methylamino]-3-nitro-benzoesäure-methylester

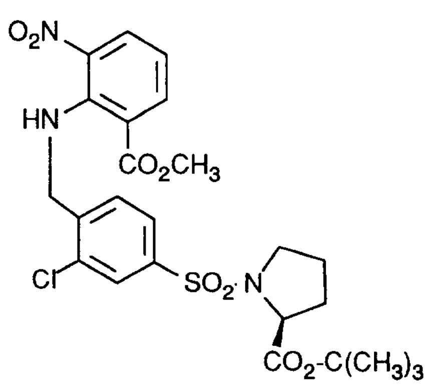

800 mg (2,8 mmol) 2-Amino-3-nitrobenzoesäure-methylester und 1,24 g (2,8 mmol) der Verbindung aus Beispiel VIII wurden mit 392 mg (2,8 mmol) $K_2CO_3$ in 10 ml Dimethylformamid für 16 h bei 20 °C gerührt. Nach wäßriger Aufarbeitung und anschließender Chromatographie der Rohprodukte an Kieselgel (PE/EE

7:3) erhielt man 760 mg (42% d.Th.) der Titelverbindung.
$R_f$: 0,40 (PE/EE 7:3)

Beispiel XII

(S)-2-[3-Fluor-1-(N-2-(tert.butoxycarbonyl)pyrrolidinyl)sulfonyl-phen-4-yl-methylamino]-3-nitrobenzoesäure-methylester

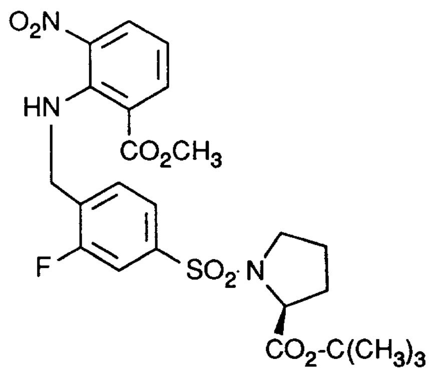

In Analogie zur Vorschrift des Beispiels XI erhielt man aus 800 mg (2,8 mmol) 2-Amino-3-nitrobenzoesäure-methylester und 1,32 g (3,1 mmol) der Verbindung aus Beispiel X 1,35 g (76% d.Th.) der Titelverbindung.
$R_f$ = 0,37 (PE/EE 7:3)

Herstellungsbeispiele

Beispiel 1

(S)-2-Butyl-1-[3-chlor-1-(N-2-tert.butoxycarbonylpyrrolidinyl)sulfonyl]-7-methoxycarbonyl-benz[d]imidazol

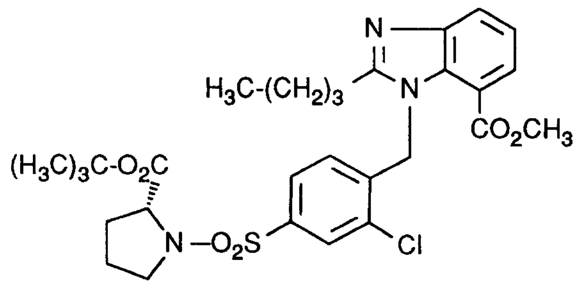

754 mg (1,18 mmol) der Verbindung aus Beispiel XI wurden mit 484 µl konz. HCL in 1.08 ml Methanol gerührt. Man gab 205 mg (3,68 mmol) Fe Pulver hinzu und erhitzte für 3 h auf 70-80 °C. Man kühlte ab, filtrierte, nahm das Filtrat in $NaHCO_3$-Lösung und Essigester auf und extrahierte nach Phasentrennung die wäßrige nach dreimal mit Essigester. Die vereinigten organischen Phasen wurden im Vakuum vom Lösemittel befreit und der Rückstand an Kieselgel chromatographiert. Man erhielt 306 mg (43% d.Th.) der Titelverbindung.
$R_f$ = 0,27 (PE/EE 7:3)

Beispiel 2

(S)-2-Butyl-1-[3-chlor-1-(N-2'-carboxy-pyrrolidinyl)-sulfonyl]-7-methoxycarbonyl-benz[d]imidazol

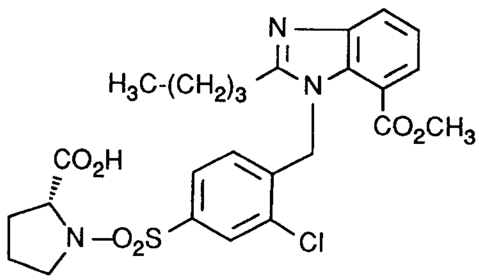

250 mg der Verbindung von Beispiel 1 wurden in 10 ml Dichlormethan gelöst und bei 0 °C mit 2 ml Trifluoressigsäure versetzt. Man rührte 3 h bei Raumtemperatur nach und zog alles Flüchtige im Vakuum ab. Man erhielt 226 mg (100% d.Th.) der Titelverbindung.
$R_f$ = 0,57 ($CH_2Cl_2$/MeOH 10:1)

Beispiel 3

(S)-2-Butyl-7-carboxy-1-[3-chlor-1-(N-2-carboxy-pyrrolidinyl)-sulfonyl]-benz[d]imidazol

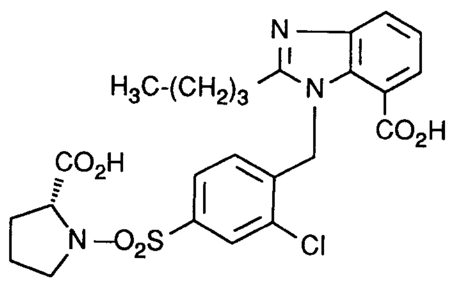

205 mg (0,38 mmol) der Verbindung aus Beispiel 2 wurden in eine Mischung aus 2 ml THF, 2 ml $H_2O$ und 0,5 ml MeOH gelöst. Nach Zugabe von 97 mg (2,3 mmol) LiOH rührte man 4 h bei RT nach. Man zog alles Flüchtige ab, nahm den Rückstand in $H_2O$ auf und extrahierte dreimal mit Essigester. Die vereinigten organischen Phasen wurden getrocknet und eingeengt. Man erhielt 176 mg (88% d.Th.) der Titelverbindung.
$R_f$ = 0,12 (Toluol/EE/$CH_3CO_2H$ 20:20:1)
Die in der Tabelle 1 aufgeführten Verbindungen wurden in Analogie zu den oben beschriebenen Beispielen hergestellt:

Tabelle 1:

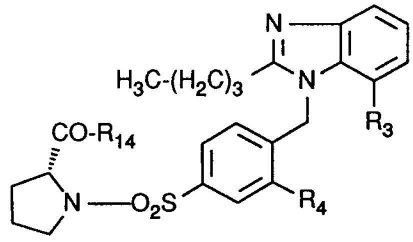

| Bsp.-Nr. | $R^3$ | R4 | $R^{14}$ | $R_f$ |
|---|---|---|---|---|
| 4 | $CO_2CH_3$ | F | $O\text{-}C(CH_3)_3$ | 0,19 PE/EE 7:3 |
| 5 | $CO_2CH_3$ | F | OH | 0,51 $CH_2Cl_2$/MeOH 10:1 |
| 6 | $CO_2H$ | F | OH | 0,12 Toluol/EE/$CH_3CO_2H$ 20:20:1 |

Beispiel 7

(S)-2-Butyl-7-carboxy-1-[3-chlor-1-(N-2-carboxypyrrolidinyl)-sulfonyl]benz[d]imidazol Bis Kaliumsalz

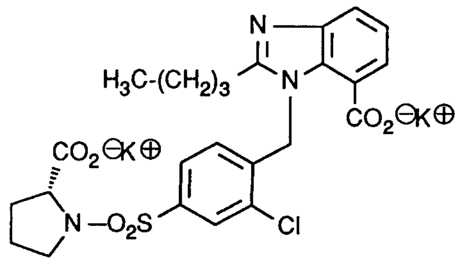

104 mg (0,2 mmol) der Verbindung von Beispiel 3 wurden in 1,5 ml Dioxan und 3,5 mi $H_2O$ gelöst, mit 0,4 ml 1 N KOH Losung versetzt und gefriergetrocknet. Man erhielt 120 mg (100% d.Th.) der Titelverbindung.

**Patentansprüche**

**1.** Benzimidazole der allgemeinen Formel (I)

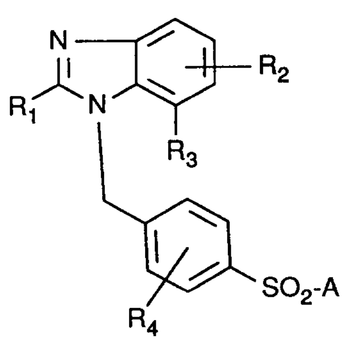

(I)

in welcher

| | |
|---|---|
| $R^1$ | für geradkettiges oder verzweigtes Alkyl mit bis zu 8 Kohlenstoffatomen steht, das gegebenenfalls durch Cycloalkyl mit 3 bis 6 Kohlenstoffatomen, Halogen, Hydroxy, Phenyl oder durch geradkettiges oder verzweigtes Alkoxy mit bis zu 6 Kohlenstoffatomen substituiert ist, oder<br>für Cycloalkyl mit 3 bis 8 Kohlenstoffatomen steht, oder<br>für eine Gruppe der Formel $-OR^5$ steht, worin |
| $R^5$ | geradkettiges oder verzweigtes Alkyl mit bis zu 8 Kohlenstoffatomen, Cycloalkyl mit 3 bis 7 Kohlenstoffatomen, Aryl mit 6 bis 10 Kohlenstoffatomen oder einen 5- bis 7-gliedrigen, gesättigten oder ungesättigten Heterocyclus mit bis zu 4 Heteroatomen aus der Reihe S, N oder O bedeutet, |
| $R^2$ | für Wasserstoff, Halogen oder für geradkettiges oder verzweigtes Alkyl oder Alkenyl mit jeweils bis zu 8 Kohlenstoffatomen steht, die gegebenenfalls durch Cycloalkyl mit 3 bis 6 Kohlenstoffatomen substituiert sind, oder<br>für Cycloalkyl mit 3 bis 8 Kohlenstoffatomen steht, oder<br>für geradkettiges oder verzweigtes Perfluoralkyl mit bis zu 6 Kohlenstoffatomen steht, |
| $R^3$ | für einen Rest der Formel<br>$-CO-R^6$ , $-SO_3H$ , |

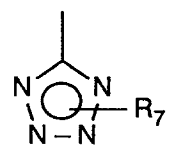

| | |
|---|---|
| | oder $-PO_3H$<br>steht, worin |
| $R^6$ | Hydroxy oder geradkettiges oder verzweigtes Alkoxy mit bis zu 8 Kohlenstoffatomen bedeutet, das gegebenenfalls durch Halogen, Carboxy, Hydroxy, Cyano oder durch geradkettiges oder verzweigtes Alkoxy oder Alkoxycarbonyl mit jeweils bis zu 6 Kohlenstoffatomen substituiert ist, oder<br>Aryloxy mit 6 bis 10 Kohlenstoffatomen oder<br>Heterocyclyloxy bedeutet, oder<br>Cycloalkyloxy mit 3 bis 7 Kohlenstoffatomen bedeutet, oder<br>eine Gruppe der Formel $-NR^8R^9$ bedeutet, worin |
| $R^8$ und $R^9$ | gleich oder verschieden sind und Wasserstoff oder geradkettiges oder verzweigtes Alkyl mit bis zu 6 Kohlenstoffatomen oder Phenyl bedeuten, |
| $R^7$ | Wasserstoff, geradkettiges oder verzweigtes Alkyl mit bis zu 6 Kohlenstoffatomen oder Triphenylmethyl bedeutet |
| $R^4$ | für Wasserstoff, Halogen, geradkettiges oder verzweigtes Alkyl mit bis zu 8 Kohlenstoffatomen steht, oder<br>für geradkettiges oder verzweigtes Perfluoralkyl mit bis zu 6 Kohlenstoffatomen steht, oder<br>für eine Gruppe der Formel $-OR^{10}$ oder $-SO_2-NR^{11}R^{12}$ steht, worin |
| $R^{10}$ | die oben angegebene Bedeutung von $R^5$ hat und mit dieser gleich oder verschieden ist, oder eine Hydroxyschutzgruppe oder Wasserstoff bedeutet, |
| $R^{11}$ und $R^{12}$ | gleich oder verschieden sind und Wasserstoff, Cycloalkyl mit 3 bis 7 Kohlenstoffatomen, Aryl mit 6 bis 10 Kohlenstoffatomen oder einen 5- bis 7-gliedrigen, gesättigten oder ungesättigten Heterocyclus mit bis zu 3 Heteroatomen aus der Reihe S, N oder O bedeuten, oder geradkettiges oder verzweigtes Alkyl mit bis zu 6 Kohlenstoffatomen bedeuten, das gegebenenfalls durch Halogen, Carboxy, Hydroxy, Cyano oder durch geradkettiges oder verzweigtes Alkoxy oder Alkoxycarbonyl mit jeweils bis zu 6 Kohlenstoffatomen substituiert ist, oder |
| $R^{11}$ und $R^{12}$ | gemeinsam unter Einbezug des Stickstoffatoms einen 5- bis 7-gliedrigen, gesättigten oder ungesättigten heterocyclischen Ring mit bis zu 3 Heteroatomen aus der Reihe S, N oder O bilden, |

A für einen über das Stickstoffatom gebundenen 3 bis 8-gliedrigen, gesättigten Heterocyclus mit bis zu 2 weiteren Heteroatomen aus der Reihe S, N oder O steht und der gegebenenfalls bis zu 2-fach gleich oder verschieden durch einen Rest der Formel

$-SO_3H$ ,

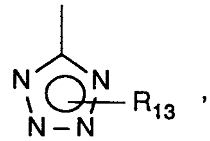

$-CO-R^{14}$ oder $-PO(OR^{15})(OR^{16})$

substituiert ist, worin

$R^{13}$ die oben angegebene Bedeutung von $R^7$ hat und mit dieser gleich oder verschieden ist und

$R^{14}$ die oben angegebene Bedeutung von $R^6$ hat und mit dieser gleich oder verschieden ist,

$R^{15}$ und $R^{16}$ gleich oder verschieden sind und Wasserstoff, geradkettiges oder verzweigtes Alkyl mit bis zu 8 Kohlenstoffatomen oder Phenyl bedeuten

und deren Salze.

**2.** Verbindungen der allgemeinen Formel (I) gemäß Anspruch 1, in welcher

$R^1$ für geradkettiges oder verzweigtes Alkyl mit bis zu 6 Kohlenstoffatomen steht, das gegebenenfalls durch Cyclopropyl, Cyclobutyl, Cyclopentyl, Cyclohexyl Fluor, Chlor, Brom, Hydroxy, Phenyl oder durch geradkettiges oder verzweigtes Alkoxy mit bis zu 4 Kohlenstoffatomen substituiert ist, oder

für Cyclopropyl, Cyclopentyl oder Cyclohexyl steht, oder

für eine Gruppe der Formel $-OR^5$ steht, worin

$R^5$ geradkettiges oder verzweigtes Alkyl mit bis zu 6 Kohlenstoffatomen, Cyclopentyl, Cyclohexyl, Cycloheptyl oder Phenyl bedeutet

$R^2$ für Wasserstoff, Halogen oder für geradkettiges oder verzweigtes Alkyl oder Alkenyl mit jeweils bis zu 6 Kohlenstoffatomen steht, die gegebenenfalls durch Cyclopropyl, Cyclobutyl, Cyclopentyl oder Cyclohexyl substituiert sind, oder

für Cyclopropyl, Cyclopentyl oder Cyclohexyl steht, oder

für geradkettiges oder verzweigtes Perfluoralkyl mit bis zu 4 Kohlenstoffatomen steht,

$R^3$ für einen Rest der Formel

$-CO-R^6$ , $-SO_3H$ ,

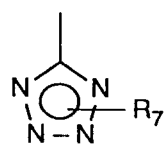

oder $-PO_3H$ steht,

worin

$R^6$ Hydroxy oder geradkettiges oder verzweigtes Alkoxy mit bis zu 6 Kohlenstoffatomen bedeutet, das gegebenenfalls durch Fluor, Chlor, Brom, Carboxy, Hydroxy, Cyano oder durch geradkettiges oder verzweigtes Alkoxy oder Alkoxycarbonyl mit jeweils bis zu 4 Kohlenstoffatomen substituiert ist, oder

Phenoxy, Cyclopentyloxy oder Cyclohexyloxy bedeutet, oder

eine Gruppe der Formel $-NR^8R^9$ bedeutet, worin

$R^8$ und $R^9$ gleich oder verschieden sind und Wasserstoff oder geradkettiges oder verzweigtes

Alkyl mit bis zu 4 Kohlenstoffatomen bedeuten,

$R^7$ Wasserstoff, geradkettiges oder verzweigtes Alkyl mit bis zu 4 Kohlenstoffatomen oder Triphenylmethyl bedeutet

$R^4$ für Wasserstoff, Fluor, Chlor, Brom, geradkettiges oder verzweigtes Alkyl mit bis zu 6 Kohlenstoffatomen, oder
für geradkettiges oder verzweigtes Perfluoralkyl mit bis zu 4 Kohlenstoffatomen steht, oder
eine Gruppe der Formel $-OR^{10}$ oder $-SO_2NR^{11}R^{12}$ steht, worin

$R^{10}$ die oben angegebene Bedeutung von $R^5$ hat und mit dieser gleich oder verschieden ist, oder Benzyl oder Wasserstoff bedeutet,

$R^{11}$ und $R^{12}$ gleich oder verschieden sind und Wasserstoff, Cyclopropyl, Cyclopentyl, Cyclohexyl, Phenyl bedeuten, oder
geradkettiges oder verzweigtes Alkyl mit bis zu 5 Kohlenstoffatomen bedeuten, das gegebenenfalls durch Fluor, Chlor, Brom, Carboxy, Hydroxy, Cyano oder durch geradkettiges oder verzweigtes Alkoxy oder Alkoxycarbonyl mit jeweils bis zu 4 Kohlenstoffatomen substituiert ist, oder

$R^{11}$ und $R^{12}$ gemeinsam unter Einbezug des Stickstoffatoms einen Morpholinring bilden,

A für über das Stickstoffatom gebundenes Azetidinyl, Piperidyl, Pyrrolidinyl oder Morpholinyl steht, die gegebenenfalls durch einen Rest der Formel
$-SO_3H$ ,

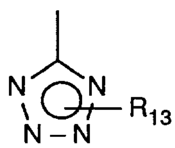 ,

$-CO-R^{14}$ oder $-PO(OR^{15})(OR^{16})$
substituiert sind, worin

$R^{13}$ die oben angegebene Bedeutung von $R^7$ hat und mit dieser gleich oder verschieden ist,

$R^{14}$ die oben angegebene Bedeutung von $R^6$ hat und mit dieser gleich oder verschieden ist,

$R^{15}$ und $R^{16}$ gleich oder verschieden sind und Wasserstoff, geradkettiges oder verzweigtes Alkyl mit bis zu 6 Kohlenstoffatomen oder Phenyl bedeuten

und deren Salze.

**3.** Verbindungen der allgemeinen Formel (I) gemäß Anspruch 1, in welcher

$R^1$ für geradkettiges oder verzweigtes Alkyl mit bis zu 4 Kohlenstoffatomen steht, das gegebenenfalls durch Fluor, Chlor, Brom, Hydroxy, Phenyl oder durch geradkettiges oder verzweigtes Alkoxy mit bis zu 3 Kohlenstoffatomen substituiert ist, oder
für Cyclopropyl, Cyclopentyl oder Cyclohexyl steht, oder
für eine Gruppe der Formel $-OR^5$ steht, worin

$R^5$ geradkettiges oder verzweigtes Alkyl mit bis zu 5 Kohlenstoffatomen, Cyclopentyl, Cyclohexyl, Cycloheptyl, Phenyl steht,

$R^2$ für Wasserstoff, Fluor, Chlor, Brom oder für geradkettiges oder verzweigtes Alkyl oder Alkenyl mit jeweils bis zu 4 Kohlenstoffatomen oder Cyclopropyl steht, oder
für geradkettiges oder verzweigtes Perfluoralkyl mit bis zu 3 Kohlenstoffatomen steht,

$R^3$ für einen Rest der Formel
$-CO-R^6$ , $-SO_3H$ ,

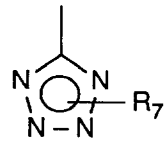

oder $-PO_3H$ steht,
worin

| | |
|---|---|
| $R^6$ | Hydroxy oder Alkoxy mit bis zu 4 Kohlenstoffatomen, Phenoxy oder Cvclopentyloxy oder Cyclohexyloxy bedeutet, oder eine Gruppe der Formel $-NR^8R^9$ bedeutet, worin |
| $R^8$ und $R^9$ | gleich oder verschieden sind und Wasserstoff oder geradkettiges oder verzweigtes Alkyl mit bis zu 3 Kohlenstoffatomen bedeuten, |
| $R^7$ | Wasserstoff, Methyl, Ethyl oder Triphenylmethyl bedeutet, |
| $R^4$ | für Wasserstoff, Fluor, Chlor, geradkettiges oder verzweigtes Alkyl mit bis zu 4 Kohlenstoffatomen, oder für geradkettiges oder verzweigtes Perfluoralkyl mit bis zu 3 Kohlenstoffatomen steht, oder für eine Gruppe der Formel $-OR^{10}$ oder $-SO_2NR^{11}R^{12}$ steht, worin |
| $R^{10}$ | die oben angegebene Bedeutung von $R^5$ hat und mit dieser gleich oder verschieden ist, oder Benzyl oder Wasserstoff bedeutet, |
| $R^{11}$ und $R^{12}$ | gleich oder verschieden sind und Wasserstoff, Cyclopropyl, Cyclopentyl, Cyclohexyl, Phenyl oder geradkettiges oder verzweigtes Alkyl mit bis zu 4 Kohlenstoffatomen bedeuten, |
| A | für über das Stickstoffatom gebundenes Azetidinyl, Piperidyl oder Pyrrolidinyl steht, die gegebenenfalls durch einen Rest der Formel $-SO_3H$ , |

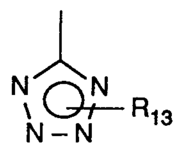 ,

$-CO-R^{14}$ oder $-PO(OR^{15})(OR^{16})$
substituiert ist, worin

| | |
|---|---|
| $R^{13}$ | die oben angegebene Bedeutung von $R^7$ hat und mit dieser gleich oder verschieden ist, |
| $R^{14}$ | die oben angegebene Bedeutung von $R^6$ hat und mit dieser gleich oder verschieden ist, |
| $R^{15}$ und $R^{16}$ | gleich oder verschieden sind und Wasserstoff, geradkettiges oder verzweigtes Alkyl mit bis zu 4 Kohlenstoffatomen oder Phenyl bedeuten |

und deren Salze.

**4.** Verfahren zur Herstellung von Benzimidazolen der allgemeinen Formel (I)

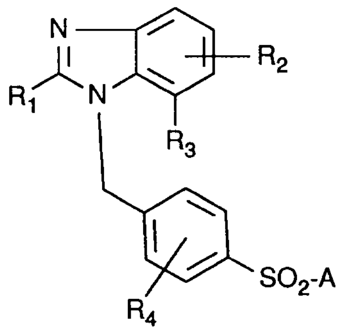

(I),

in welcher

$R^1$ für geradkettiges oder verzweigtes Alkyl mit bis zu 8 Kohlenstoffatomen steht, das gegebenenfalls durch Cycloalkyl mit 3 bis 6 Kohlenstoffatomen, Halogen, Hydroxy, Phenyl oder durch geradkettiges oder verzweigtes Alkoxy mit bis zu 6 Kohlenstoffatomen substituiert ist, oder
für Cycloalkyl mit 3 bis 8 Kohlenstoffatomen steht, oder
für eine Gruppe der Formel $-OR^5$ steht, worin

$R^5$ geradkettiges oder verzweigtes Alkyl mit bis zu 8 Kohlenstoffatomen, Cycloalkyl mit 3 bis 7 Kohlenstoffatomen, Aryl mit 6 bis 10 Kohlenstoffatomen oder einen 5- bis 7-gliedrigen, gesättigten oder ungesättigten Heterocyclus mit bis zu 4 Heteroatomen aus der Reihe S, N oder O bedeutet,

$R^2$ für Wasserstoff, Halogen oder für geradkettiges oder verzweigtes Alkyl oder Alkenyl mit jeweils bis zu 8 Kohlenstoffatomen steht, die gegebenenfalls durch Cycloalkyl mit 3 bis 6 Kohlenstoffatomen substituiert sind, oder
für Cycloalkyl mit 3 bis 8 Kohlenstoffatomen steht, oder
für geradkettiges oder verzweigtes Perfluoralkyl mit bis zu 6 Kohlenstoffatomen steht,

$R^3$ für einen Rest der Formel
$-CO-R^6$ , $-SO_3H$ ,

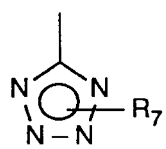

oder $-PO_3H$
steht, worin

$R^6$ Hydroxy oder geradkettiges oder verzweigtes Alkoxy mit bis zu 8 Kohlenstoffatomen bedeutet, das gegebenenfalls durch Halogen, Carboxy, Hydroxy, Cyano oder durch geradkettiges oder verzweigtes Alkoxy oder Alkoxycarbonyl mit jeweils bis zu 6 Kohlenstoffatomen substituiert ist, oder
Aryloxy mit 6 bis 10 Kohlenstoffatomen oder Heterocyclyloxy bedeutet, oder
Cycloalkyloxy mit 3 bis 7 Kohlenstoffatomen bedeutet, oder eine Gruppe der Formel $-NR^8R^9$ bedeutet, worin

$R^8$ und $R^9$ gleich oder verschieden sind und Wasserstoff oder geradkettiges oder verzweigtes Alkyl mit bis zu 6 Kohlenstoffatomen oder Phenyl bedeuten,

$R^7$ Wasserstoff, geradkettiges oder verzweigtes Alkyl mit bis zu 6 Kohlenstoffatomen oder Triphenylmethyl bedeutet

$R^4$ für Wasserstoff, Halogen, geradkettiges oder verzweigtes Alkyl mit bis zu 8 Kohlenstoffatomen steht, oder
für geradkettiges oder verzweigtes Perfluoralkyl mit bis zu 6 Kohlenstoffatomen steht, oder

für eine Gruppe der Formel $-OR^{10}$ oder $-SO_2-NR^{11}R^{12}$ steht, worin

$R^{10}$ die oben angegebene Bedeutung von $R^5$ hat und mit dieser gleich oder verschieden ist, oder eine Hydroxyschutzgruppe oder Wasserstoff bedeutet,

$R^{11}$ und $R^{12}$ gleich oder verschieden sind und Wasserstoff, Cycloalkyl mit 3 bis 7 Kohlenstoffatomen, Aryl mit 6 bis 10 Kohlenstoffatomen oder einen 5- bis 7-gliedrigen, gesättigten oder ungesättigten Heterocyclus mit bis zu 3 Heteroatomen aus der Reihe S, N oder O bedeuten, oder geradkettiges oder verzweigtes Alkyl mit bis zu 6 Kohlenstoffatomen bedeuten, das gegebenenfalls durch Halogen, Carboxy, Hydroxy, Cyano oder durch geradkettiges oder verzweigtes Alkoxy oder Alkoxycarbonyl mit jeweils bis zu 6 Kohlenstoffatomen substituiert ist, oder

$R^{11}$ und $R^{12}$ gemeinsam unter Einbezug des Stickstoffatoms einen 5- bis 7-gliedrigen, gesättigten oder ungesättigten heterocyclischen Ring mit bis zu 3 Heteroatomen aus der Reihe S, N oder O bilden,

A für einen über das Stickstoffatom gebundenen 3 bis 8-gliedrigen, gesättigten Heterocyclus mit bis zu 2 weiteren Heteroatomen aus der Reihe S, N oder O steht und der gegebenenfalls bis zu 2-fach gleich oder verschieden durch einen Rest der Formel

$-SO_3H$ ,

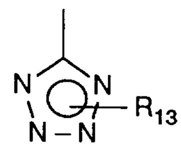 ,

$-CO-R^{14}$ oder $-PO(OR^{15})(OR^{16})$

substituiert ist, worin

$R^{13}$ die oben angegebene Bedeutung von $R^7$ hat und mit dieser gleich oder verschieden ist und

$R^{14}$ die oben angegebene Bedeutung von $R^6$ hat und mit dieser gleich oder verschieden ist,

$R^{15}$ und $R^{16}$ gleich oder verschieden sind und Wasserstoff, geradkettiges oder verzweigtes Alkyl mit bis zu 8 Kohlenstoffatomen oder Phenyl bedeuten

und deren Salze,
dadurch gekennzeichnet, daß man
Sulfonylbenzylverbindungen der allgemeinen Formel (II)

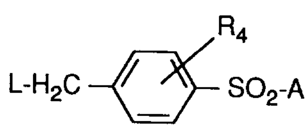 (II)

in welcher

A und $R^4$ die oben angegebene Bedeutung haben und

L für Halogen steht,

mit Nitrobenzoesäureestern der allgemeinen Formel (III)

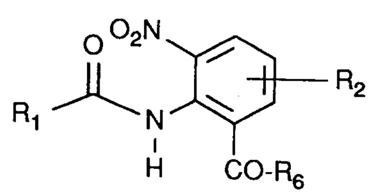 (III)

in welcher

$R^1$, $R^2$ und $R^6$ die oben angegebene Bedeutung haben,

zunächst in inerten Lösemitteln, in Anwesenheit einer Base umsetzt, und anschließend in Gegenwart von Eisenpulver und Säure cyclisiert und gegebenenfalls die Substituenten $R^1$, $R^2$, $R^3$ und $R^4$ nach üblichen Methoden variiert.

**5.** Verbindungen der allgemeinen Formel (I) gemäß Anspruch 1 zur Verwendung bei der Bekämpfung von Erkrankungen.

**6.** Arzneimittel enthaltend mindestens eine Verbindung der allgemeinen Formel (I) gemäß Anspruch 1.

**7.** Verfahren zur Herstellung von Arzneimitteln, dadurch gekennzeichnet, daß man mindestens eine Verbindung der allgemeinen Formel (I) gemäß Anspruch 1 gegebenenfalls unter Verwendung von üblichen Hilfs- und Trägerstoffen in eine geeignete Applikationsform überführt.

**8.** Verwendung von Verbindungen der allgemeinen Formel (I) gemäß Anspruch 1 bei der Herstellung von Arzneimitteln zur Bekämpfung von Kreislauf- und Gefäßerkrankungen.